# EUROPEAN PATENT APPLICATION

(11) **EP 4 385 465 A1**
(43) Date of publication of application: **19.06.2024**
(21) Application number: 23217209.8
(22) Date of filing: 15.12.2023
(51) Int. Cl.: A61F 2/95, A61F 2/24, A61F 2/962

(54) **DELIVERY DEVICE WITH AN ACTUATOR HAVING A HARD STOP AND RELATED SYSTEMS AND METHODS**

(30) Priority: 15.12.2022 AU 2022903862
(71) Applicant: Cook Medical Technologies LLC, Bloomington, IN 47404 (US)
(72) Inventor: Collins, James T., Queensland, 4064 (AU); Smith, Logan R. M., Queensland, 4122 (AU); Burrell, John, Ohio, 45040 (US)
(74) Representative: Williams Powell

(57) **Abstract**

Delivery device assemblies useful in the delivery of implantable medical devices, such as endografts, into a vascular system are described. A delivery device has a handle (200) having a handle body (220), at least one actuator (310) mounted to the handle body (220) for rotation about the handle body, the actuator having a follower-driver (322). For each actuator (310), at least one follower (510) is disposed around the handle body (220) and is configured to rotate about the handle body. A hard stop (218) on the handle body (220) arrests rotation of a distal most follower for each actuator (310). Delivery systems, methods of making a delivery device, methods of making a delivery system, and methods of delivering an implantable medical device to a point of treatment in a body lumen are also described.

## Description

### FIELD

The disclosure relates to medical devices. More particularly, the disclosure relates to delivery devices useful in the delivery of implantable medical devices, such as endografts and other prostheses, into a body lumen of a patient, such as a human being or other animal. Specific examples described in the disclosure relate to delivery devices, delivery systems, methods of making a delivery device, methods of making a delivery system, and methods of delivering an implantable medical device to a point of treatment in a body lumen.

### BACKGROUND

The use of delivery devices, introducers, catheters, and other medical devices is known for a variety of medical procedures, including procedures for establishing, reestablishing or maintaining passages, cavities or lumens in vessels, organs or ducts in human and veterinary patients, occlusion of such vessels, delivering medical treatments, and other interventions.

Stent grafts and delivery devices are used in a variety of aortic interventional treatments. For example, interventionalists frequently use delivery devices to implant stent grafts to treat aortic aneurysms and to repair regions of the aorta, including the aortic arch, the thoracic aorta, the abdominal aorta, and the aortic bifurcation. Use of a delivery device in these procedures allows an interventionalist to deliver an implantable medical device, such as an endograft or other intraluminal medical device, to a specific point of treatment in a body lumen of a patient from a remote location.

Numerous devices and procedures have been developed that involve the percutaneous insertion of a prosthesis into a body lumen, such as a blood vessel or duct, of a patient. Such a prosthesis may be introduced into the lumen by a variety of known techniques. For example, a wire guide may be introduced into a blood vessel using the Seldinger technique. This technique involves creating a surgical opening in the vessel with a needle and inserting a wire guide into the vessel through a bore of the needle. The needle can be withdrawn, leaving the wire guide in place. A delivery device is then inserted over the wire guide and into the vessel. The delivery device may be used in conventional fashion to insert into the blood vessel a variety of implantable medical devices, such as stents, stent grafts, including endografts, catheters, cardiac leads, balloons, and the like.

For example, the delivery device may be used to deliver and deploy an expandable support frame, such as a stent graft, to an aneurysmal blood vessel site. A stent graft is usually formed from a tubular body of a biocompatible graft material with one or more stents mounted into or onto the tubular body to provide support therefor. The stents may be balloon expandable stents and/or self-expanding stents. The deployment of the prosthesis into the lumen of a patient from a remote location by the use of a delivery device is described in, for example, U.S. Pat. No. 7,435,253 to Hartley entitled *"*A Prosthesis and a Method and Means of Deploying a Prosthesis", which is incorporated herein by reference in its entirety.

Some delivery devices include annular reels which are rotated by an interventionalist during delivery of an associated implantable medical device into a body lumen of a patient. For example, annular reels may be used with actuators for removing ties that maintain a reduced diameter of the implantable medical device within the delivery system, for releasing one or both ends of the implantable medical device from the delivery system, and for other functions. Annular reels make it difficult, however, for an interventionalist to know how far to rotate an associated actuator with respect to a handle of the delivery device to achieve a desired result, such as removal of diameter-reducing ties and release of one or both ends of the implantable medical device, as mentioned above. Insufficient or excessive rotation of an actuator in a delivery device can be undesirable as either may leave an associated function incomplete.

A need remains, therefore, for new and improved delivery devices useful in the delivery of implantable medical devices, delivery systems, methods of making a delivery device, methods of making a delivery system, and methods of delivering an implantable medical device to a point of treatment in a body lumen.

### BRIEF SUMMARY OF SELECTED EXAMPLES

Various example delivery devices, delivery systems, methods of making a delivery device, methods of making a delivery system, and methods of delivering an implantable medical device to a point of treatment in a body lumen are described.

According to an aspect of the disclosure, there is provided a delivery device assembly for endo-luminal surgery, the delivery device assembly comprising: a handle assembly having a handle body; a hand-graspable actuator mounted to the handle body for rotation about the handle body, the actuator having a follower-driving portion; a plurality of followers disposed axially adjacent to each other around a shaft portion of the body, each follower comprising an arcuate body portion terminating in circumferentially spaced-apart first and second ends, the first and second ends resiliently displaceable from each other to allow a snap-fit over the shaft portion; and a hard stop fixed against rotation with respect to the handle body, wherein the follower-driving portion, the plurality of followers and the hard stop act together to arrest rotation of the actuator with respect to the handle body at a total rotation of X degrees, where X is greater than 360 degrees.

Advantageously, each follower further comprises a lug portion extending radially outwards from the lug body portion, the lug portion having an axially extending cantilever portion, the cantilever portion nestable over the lug body portion of an adjacent follower of the plurality of followers.

Preferably, rotation of the actuator with respect to the handle body is arrested by sandwiching of the lug portions of the followers between the follower-driving portion of the actuator and the hard stop.

Advantageously, each lug portion has a first face and a second face, the second face on the cantilever portion, wherein the second face rotates around the shaft portion as the first face is pushed around the shaft portion.

Preferably, the total rotation is: (360 - y) * z, where y = the lug arc in degrees and z = the number of followers.

Advantageously, an arcuate distance between the first face and the second face defines a lug arc.

Preferably, rotation of the actuator induces rotation of a first of the plurality of followers though a first follower neutral arc, followed by rotation of the first follower through a first follower working arc, during which a second follower of the plurality of followers is rotated by the first follower, wherein at an end of a final working arc, the second follower or a subsequent follower, engages the hard stop thereby stopping any further rotation of the actuator.

Advantageously, each follower comprises an arcuate bearing surface engaged with the shaft portion for relative rotation.

Preferably, the bearing surface extends around a bearing arc of at least 190 degrees around the shaft portion.

Advantageously, the actuator actuates a line puller.

Preferably, the line puller retracts diameter reducing ties within a stent-graft.

According to another aspect of the disclosure, there is provided a delivery device assembly for endo-luminal surgery, the delivery device assembly having a positioner extending from a handle assembly towards a prosthesis receiving zone and a tip, the assembly comprising: a hand-graspable actuator mounted to a handle body for rotation about the handle body, the actuator having a follower-driving portion; a plurality of followers disposed axially adjacent to each other around a shaft portion of the body, each follower comprising an arcuate body portion terminating in circumferentially spaced-apart first and second ends, the first and second ends resiliently displaceable from each other to allow a snap-fit over the shaft portion; and a hard stop fixed against rotation with respect to the handle body, wherein the follower-driving portion, the plurality of followers and the hard stop act together to arrest rotation of the actuator with respect to the handle body at a total rotation of X degrees, where X is greater than 360 degrees.

An example delivery device comprises a handle assembly having a lengthwise axis and comprising: a proximal handle portion; a distal handle portion having a distal handle body defining a circumferential channel and a hard stop adjacent the circumferential channel; a plurality of followers disposed within the circumferential channel and individually rotatable about the distal handle body, each follower of the plurality of followers having a follower body and a lug having an axially extending cantilever portion with a first face and a second face, the cantilever portion of a first follower of the plurality of followers nestable over the follower body of an adjacent second follower of the plurality of followers such that a second face of the first follower of the plurality of followers contacts a first face of the second follower of the plurality of followers upon rotation of the first follower about the distal handle body; and an actuator disposed over the plurality of followers and rotatable about the distal handle body, the actuator defining a follower driver extending radially inward toward the distal handle body and configured to contact the first face of the first follower of the plurality of followers such that rotation of the actuator drives rotation of the first follower of the plurality of followers; an inner elongate member extending from the handle assembly; and a sheath disposed over the inner elongate member.

Another example delivery device comprises a handle assembly having a lengthwise axis and comprising: a proximal handle portion defining a rounded triangular cross-sectional shape; a distal handle portion defining a circular cross-sectional shape, a first circumferential wall defining a hard stop, and a second circumferential wall, a circumferential channel disposed between the first and second circumferential walls; a first follower disposed in the circumferential channel, the first follower rotatable about the distal handle body and defining a first follower body and a first follower lug having a first follower cantilever portion with a first face and a second face, the first follower cantilever portion extending axially over the first circumferential wall such that the second face of the first follower cantilever portion is configured to contact the hard stop following rotation of the first follower; a second follower disposed in the circumferential channel, the second follower rotatable about the distal handle body and defining a second follower body and a second follower lug having a second follower cantilever portion with a first face and a second face, the second follower cantilever portion extending axially over the first follower body such that the second face of the second follower cantilever portion is configured to contact the first face of the first follower cantilever portion following rotation of the first follower; and an actuator disposed over the plurality of followers and rotatable about the distal handle body, the actuator defining a follower driver extending radially inward toward the distal handle body and configured to contact the first face of the first follower of the plurality of followers such that rotation of the actuator drives rotation of the first follower of the plurality of followers; an inner elongate member extending from the handle assembly; and a sheath disposed over the inner elongate member.

Another example delivery device comprises a handle assembly having a lengthwise axis and comprising: a proximal handle portion defining a rounded triangular cross-sectional shape; a distal handle portion defining a circular cross-sectional shape, a circumferential channel, and a hard stop adjacent the circumferential channel; a follower disposed in the circumferential channel and rotatable about the distal handle body, the follower defining a cantilever portion extending axially over the first circumferential wall configured to contact the hard stop; and an actuator disposed over the follower and rotatable about the distal handle body, the actuator defining a follower driver extending radially inward toward the distal handle body and configured to contact the follower such that rotation of the actuator drives rotation of the follower; an inner elongate member extending from the handle assembly; and a sheath disposed over the inner elongate member.

An example delivery system comprises a delivery device having a handle assembly having a lengthwise axis and comprising: a proximal handle portion defining a rounded triangular cross-sectional shape; a distal handle portion defining a circular cross-sectional shape, a circumferential channel, and a hard stop adjacent the circumferential channel; a follower disposed in the circumferential channel and rotatable about the distal handle body, the follower defining a cantilever portion extending axially over the first circumferential wall configured to contact the hard stop; and an actuator disposed over the follower and rotatable about the distal handle body, the actuator defining a follower driver extending radially inward toward the distal handle body and configured to contact the follower such that rotation of the actuator drives rotation of the follower; an inner elongate member extending from the handle assembly; an implantable medical device disposed on the inner elongate member; and a sheath disposed over the inner elongate member and the implantable medical device.

An example method of making a delivery device comprises determining a desired total number of actuators; for an individual actuator in the desired total number of actuators, determining a desired total range of rotation for the individual actuator; selecting a group of followers having suitable lug arc and axial length values to provide the desired total range of rotation of the individual actuator; disposing each follower of the group of followers in a channel of a handle body portion; assembling an inner shell over the group of followers; and assembling an outer shell over the inner shell to provide a completed actuator.

An example method of making a delivery system comprises disposing an implantable medical device about an elongate member of a delivery device according to an embodiment.

An example method of delivering an implantable medical device to a point of treatment in a body lumen comprises navigating an end of a delivery system according to an embodiment to a point of treatment in a body lumen; and rotating an individual actuator of a group of actuators in a delivery device of a delivery system according to an embodiment until rotation of the actuator is arrested.

### DESCRIPTION OF FIGURES

Additional understanding of these and other example delivery devices, delivery systems, methods of making a delivery device, methods of making a delivery system, and methods of delivering an implantable medical device to a point of treatment in a body lumen can be obtained by review of the brief summary of selected examples, above, the detailed description of selected examples, below, and the referenced drawings, in which:
Figure 1A is a perspective view of an example delivery system having an implantable medical device disposed on a delivery device.
Figure 1B is a perspective view of the delivery system illustrated in Figure 1A. The sheath of the delivery device is retracted to expose the implantable medical device.
Figure 1C is a perspective view of an alternative yoke shown in the environment of a delivery system.
Figure 1D is a front view of the alternative yoke illustrated in Figure 1C.
Figure 2 is an exploded perspective view of a portion of the delivery device of the delivery system illustrated in Figure 1.
Figure 3A is a perspective view of a portion of the delivery device of the delivery system illustrated in FIGURE 1. A second half of an inner shell component of the delivery system is shown separated from the delivery system and inverted.
Figure 3B is a magnified perspective view of a portion of the delivery device of the delivery system illustrated in FIGURE 1. Three followers are illustrated for an actuator of the delivery device.
Figure 3C is a magnified perspective view of a portion of the delivery device of the delivery system illustrated in FIGURE 1. A single alternative follower is illustrated for an actuator of the delivery device.
Figure 3D is a magnified perspective view of a portion of the delivery device of the delivery system illustrated in FIGURE 1. Two alternative followers are illustrated for an actuator of the delivery device.
Figure 4 is a plan view of a follower of the delivery device of the delivery system illustrated in Figure 1.
Figure 5 is a front view of the follower illustrated in Figure 4.
Figure 6 is a side view of the follower illustrated in Figure 4.
Fig 7A is a perspective view of the follower illustrated in Figure 4.
Figure 7B is a perspective view of the follower illustrated in Figure 4.
Figure 8A is a partial front view of two followers of the delivery device of the delivery system illustrated in Figure 1. The arrow shows a direction of rotation.
Figure 8B is a partial top view of the two followers illustrated in Figure 8A. The arrow shows a direction of rotation.
Figure 9A is a partial perspective view of the delivery device of the delivery system illustrated in Figure 1. Three followers are shown in an initial configuration. The arrow shows a direction of rotation.
Figure 9B is a perspective view of the portion of the delivery device illustrated in Figure 9A. The three followers are shown in a configuration resulting from rotation of a follower in the direction indicated by the arrow.
Figure 9C is a perspective view of the portion of the delivery device illustrated in Figure 9A. The three followers are shown in a configuration resulting from further rotation of a follower in the direction indicated by the arrow.
Figure 9D is a perspective view of the portion of the delivery device illustrated in Figure 9A. The three followers are shown in a configuration resulting from further rotation of a follower in the direction indicated by the arrow.
Figure 9E is a perspective view of the portion of the delivery device illustrated in Figure 9A. The three followers are shown in a configuration resulting from further rotation of a follower in the direction indicated by the arrow.
Figure 9F is a perspective view of the portion of the delivery device illustrated in Figure 9A. The three followers are shown in a configuration resulting from further rotation of a follower in the direction indicated by the arrow.
Figure 9G is a perspective view of the portion of the delivery device illustrated in Figure 9A. The three followers are shown in a configuration resulting from further rotation of a follower in the direction indicated by the arrow.
Figure 10A is a partial sectional view illustrating the follower illustrated in Figure 4 relative to an inner handle body of the delivery device of the delivery system illustrated in Figure 1A. Arrows indicate direction of manipulations of the follower to snap fit the follower onto the inner handle body.
Figure 10B is a partial sectional view illustrating the follower illustrated in Figure 4 disposed on an inner handle body of the delivery device of the delivery system illustrated in Figure 1A. The follower is snap fit onto the inner handle body as a result of manipulations of the follower in the directions indicated by the arrows in Figure 10A.
Figure 11 is a front view of the follower illustrated in Figure 4. The follower is illustrated in an open configuration resulting from manipulations of the follower.
Figure 12 is a front view of an alternative follower.
Figure 13 is a flowchart illustration of an example method of making a delivery device.
Figure 14 is a flowchart illustration of an example method of making a delivery system.
Figure 15 is a flowchart illustration of an example method of delivering an implantable medical device to a point of treatment in a body lumen.

### DETAILED DESCRIPTION OF SELECTED EXAMPLES

The following detailed description and the appended drawings describe and illustrate various example delivery devices, delivery systems, methods of making a delivery device assembly, methods of making a delivery system, and methods of delivering an implantable medical device to a point of treatment in a body lumen in a body lumen. The description and illustration of these examples enable one skilled in the art to make and use example delivery devices and delivery systems, and to perform example methods of making a delivery device assembly, methods of making a delivery system, and methods of delivering an implantable medical device to a point of treatment in a body lumen. The described and illustrated examples do not limit the scope of the claims in any manner.

As used herein, the term "distal" refers to an end or portion of a referenced object, such as the aorta, a delivery device, or an implantable medical device, that is further away from the heart than another end or portion of the referenced object with respect to the direction of blood flow from the heart.

As used herein, the term "proximal" refers to an end or portion of a referenced object, such as the aorta, a delivery device, or an implantable medical device, that is nearer to the heart than another end or portion of the referenced object with respect to the direction of blood flow from the heart.

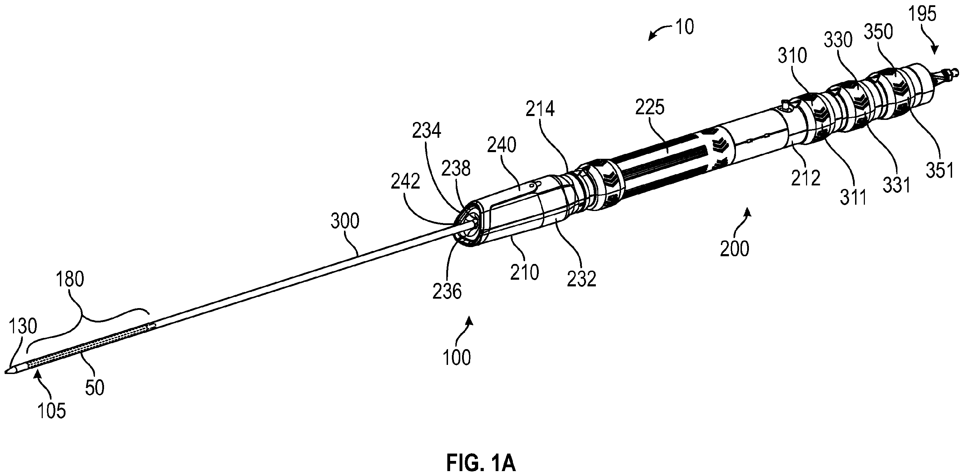

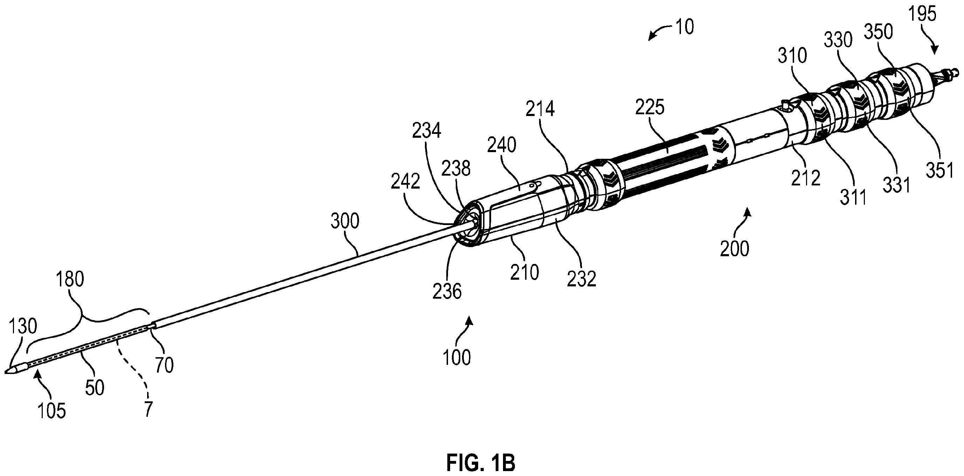

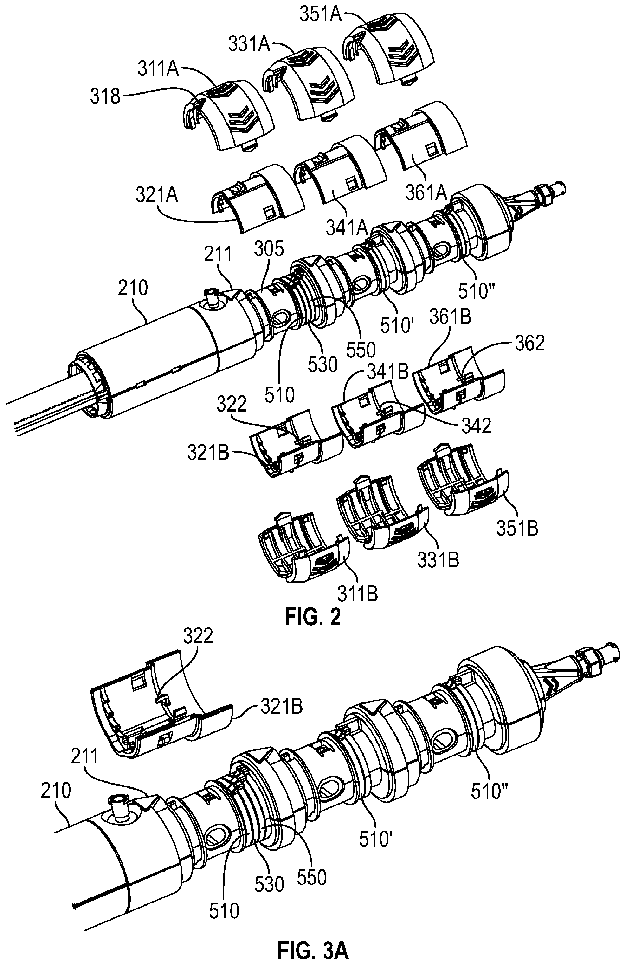

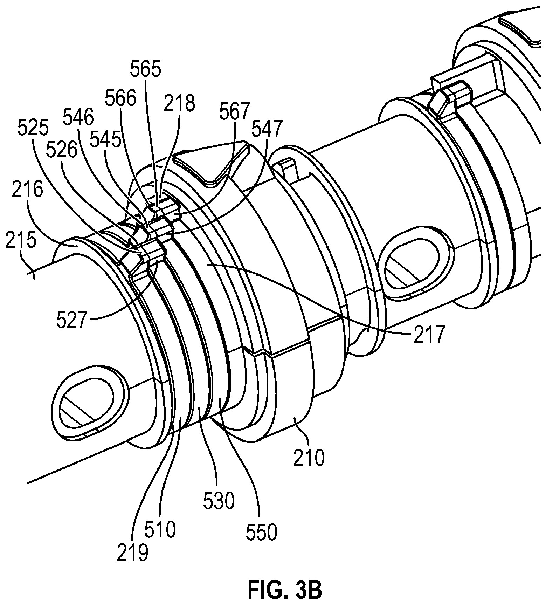

Each of Figures 1A and 1B illustrates an example delivery system 10 that includes a delivery device 100 loaded with an implantable medical device 50. Each of Figures 2, 3A, and 3B illustrate a portion of the delivery device 100. The delivery device 100 includes at least one actuator having a hard stop and is useful in the delivery of an implantable medical device, such as implantable medical device 50, into a body lumen of a patient, such as a human being or other animal. Figure 1A shows the delivery system 10 in a first configuration, in which sheath 300 is disposed over implantable medical device 50, while Figure 1B shows the delivery system 10 in a second configuration, in which sheath 300 has been retracted to expose implantable medical device 50 to the external environment. The first configuration is generally used during navigation of the delivery device through a body lumen to a point of treatment; the second configuration is generally used during deployment of the implantable medical device from the delivery system to achieve implantation of the implantable medical device at the point of treatment.

Referring to Figures 1A and 1B, Delivery device 100 has a handle assembly 200 having a proximal handle portion 210, a distal handle portion 212, and transition portion 214 axially disposed between the proximal handle portion 210 and the distal handle portion 212. First 310, second 330, and third 350 actuators are mounted to and rotatable about the distal handle body 220, as described in detail below. In use, an operator can conveniently hold the proximal handle portion 210 while rotating one of the actuators 310, 330, 350 to perform a function associated with the actuator 310, 330, 350. While delivery device 100 includes three actuators 310, 330, 350, it is noted that a delivery device does not require three actuators and, indeed, any suitable number of actuators can be used in a delivery device according to a particular embodiment. Examples of suitable numbers of actuators include, but are not limited to, one, two, a plurality, at least two, more than two, three, and more than three. Structure described herein as associated with an actuator is scaled accordingly with the number of actuators. The delivery device 100 has a proximal end 105, a distal end 195, and a prosthesis receiving zone 180. As best illustrated in FIGURE 1B, an implantable medical device 50, such as an endograft, is disposed on an inner elongate member 70 of the delivery device 100. Together, delivery device 100 and loaded implantable medical device 50 comprise delivery system 10. In Figure 1A, the prosthesis receiving zone 180 and the implantable medical device 50 are covered by a sheath 300. The delivery device 100 further comprises a tip 130 at the proximal end 105, a guide wire cannula 7, shown in Figure 1B, extending distally from the tip 130 to handle assembly 200 at the distal end 195. The guide wire cannula 7 is slidable over a guide wire 4 (not shown) to facilitate navigation of the inner elongate member 70, implantable medical device 50, and sheath 300 within a body lumen to position implantable medical device 50 at a desired point of treatment within the body lumen. A positioner (not illustrated in the figures) is disposed around the guide wire cannula 7 and extends through the sheath 300. The positioner extends from the handle assembly 200 toward the tip 130. The sheath 300 extends from the handle assembly 200 over the positioner toward the tip 130.

Proximal handle portion 210 includes a proximal handle body 232 and a yoke 234 disposed on proximal handle body 232. The proximal end of yoke 234 defines a substantially triangular-shaped opening having rounded corners, with an apron 236 that extends to inner opening 238 through which sheath 300 and inner elongate member 70 extend. Yoke 234 also includes tab 240 that extends distally and is seated in a recess defined by proximal handle portion 210. In the embodiment illustrated in Figures 1A and 1B, tab defines a valve port, which can be used for flushing, for example. Proximal handle portion 210 defines a rounded triangle cross-sectional shape, providing three substantially flat sides that facilitate gripping of the proximal handle portion 210 during use, which aids in rotation of actuators 310, 330, 350. Also, the rounded triangle cross-sectional shape of proximal handle portion 210 gives a user a tactile indication of a starting point prior to initiating rotation of any of the actuators 310, 330, 350. Furthermore, the rounded triangle cross-sectional shape of proximal handle portion 210 may prevent rolling of the handle assembly 200 when rested on a surface, such as an uneven surface.

Yoke 234 has proximal end 242 that lies on a plane disposed at a non-orthogonal angle to a lengthwise axis of delivery device 100. In Figures 1A and 1B, delivery device 100 and handle assembly 200 have the same lengthwise axis, and proximal end 242 extends distally when proceeding from the bottom of the figure to the top of the figure. This structural configuration is advantageous at least because it reduces the angle, with respect to a lengthwise axis of delivery device 100, needed for a line of sight to visualize the point at which delivery device 100 enters an opening in a body of a patient, such as a cutdown that provides access to a body lumen. This removes the need for an interventionalist to move toward the point at which delivery device 100 enters the opening, which may require loosening or release of a grip on the handle assembly 100.

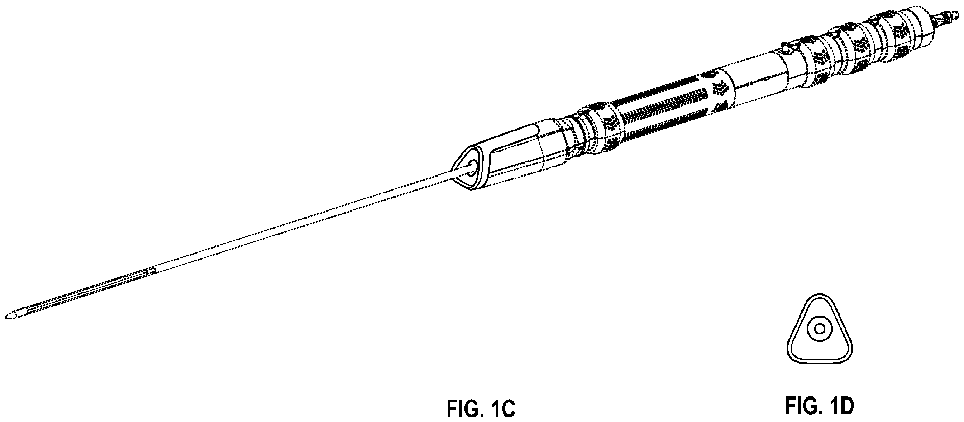

In the illustrated example, yoke 234 is a relatively flexible material, compared to the relatively rigid material of the proximal handle body 232. Furthermore, the apron 236 defines a multifaceted surface. Figures 1C and 1D illustrate an alternative yoke that having an apron that lacks the facets of yoke 234.

Distal handle body 220 defines a circular cross-sectional shape and includes grip 225. In use, a user, such as an interventionalist, holds the proximal handle portion 210 while rotating one of the actuators 310, 330, 350 to perform a function associated with the actuator 310, 330, 350 being rotated. If desired, when not rotating an actuator 310, 330, 350 or otherwise manipulating the handle assembly 200, the use can set the handle assembly 200 on a surface such that one of the three substantially flat surfaces of the proximal handle portion 210 contacts the surface on which the handle assembly 200 is positioned, allowing the proximal handle portion 210 to resist rotation of the handle assembly 200.

As best illustrated in Figure 2, each of the actuators 310, 330, and 350 includes an outer shell having first and second portions. Thus, first actuator 310 includes outer shell 311 having a first portion 311A and a second portion 311B that snap together to form outer shell 311. Second actuator 330 includes outer shell 331 having a first portion 331A and a second portion 331B that snap together to form outer shell 331. Third actuator 350 includes outer shell 351 having a first portion 351A and a second portion 351B that snap together to form outer shell 351. Also as best illustrated in Figure 2, each of the actuators 310, 330, and 350 includes an inner shell having first and second portions. Thus, first actuator 310 includes an inner shell having a first portion 321A and a second portion 321B that snap together to form the inner shell of the first actuator 310. Second actuator 330 includes an inner shell having a first portion 341A and a second portion 341B that snap together to form the inner shell of the second actuator 330. Third actuator 350 includes an inner shell having a first portion 361A and a second portion 361B that snap together to form the inner shell of the third actuator 350. In each actuator 310, 330, 350, the respective inner shell is disposed circumferentially around the inner handle body 305 of the handle assembly 200 and the respective outer shell 311, 331, 351 is disposed circumferentially around the respective inner shell.

In the illustrated example, each outer shell 311, 331, 351 includes a recess or notch that engages with a structural feature of the handle assembly to provide a locked condition where rotation is prevented, and, an unlocked condition where rotation can occur. For example, outer shell 311 includes notch 318 that engages with projection 211 on proximal handle portion 210 to prevent rotation of outer shell 311 and, indeed, or actuator 310. Distally-directed axial movement of outer shell 311 moves notch 318 away from projection 211, allowing outer shell 311 to rotated.

As best illustrated in Figure 2, each of the second portions 321B, 341B, 361B of the inner shells includes a follower-driver. Thus, second portion 321B of the inner shell of the first actuator 310 includes follower driver 322. Second portion 341B of the inner shell of the second actuator 330 includes follower driver 342. Second portion 361B of the inner shell of the third actuator 350 includes follower driver 362. Each of the follower drivers 322, 342, 362 is a projection that extends inwardly from the inner surface of the respective inner shell. As best illustrated in Figure 3A, which shows only the second portion 321B of the inner shell of the first actuator 311, follower driver 322 provides structure for contacting and driving a first follower 510 of the first actuator 310. Rotation of outer shell 311 rotates inner shell portions 321A, 321B and follower driver 322. As detailed below, through contact between follower driver 322 and a lug portion of the first follower 510, continued rotation of outer shell 311 rotates the first follower 510, and eventually the second 530 and third 550 followers.

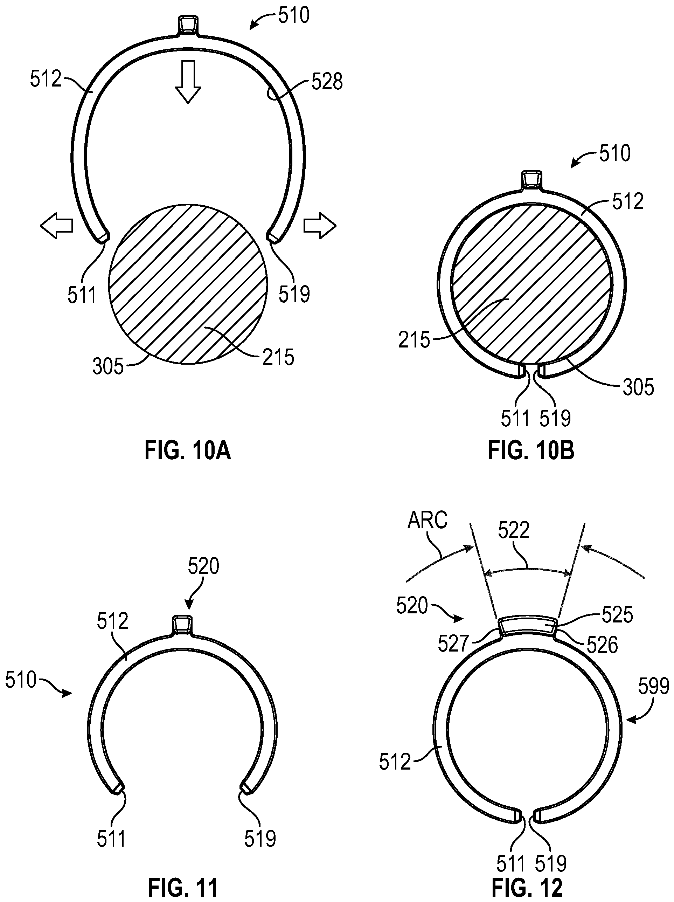

Referring to Figure 3B, a plurality of followers 510, 530, 550 is disposed around inner handle body 305 of the handle assembly 200. The followers 510, 530, 550 are positioned axially adjacent each other in a channel 219 positioned between first 215 and second 217 circumferential walls. Each of Figures 10A and 10B illustrate one follower 510 relative to the inner handle body 305. Figure 10B illustrates the follower 510 disposed around inner handle body 305, resulting from manipulations of the follower 510 in the directions of the arrows in Figure 10A, which results in a snap fit of the follower 510 onto the shaft of the inner handle body 305. The snap fit securement to the inner handle body 305 and the circumferential walls 215, 217 hold the followers 510, 530, 550 captive in channel 219, allowing the followers 510, 530, 550 to rotate about inner handle body 305 within the channel 219 during use of the delivery device 100.

The illustrated example includes three followers 510, 530, 550. Any suitable number of followers can be included, however, and a skilled artisan will be able to select an appropriate number of followers for inclusion in a delivery device according to a particular embodiment based on various considerations, including any function performed by the actuator with which the follower(s) are to be associated and the number of rotations about the inner handle body of the delivery system desired to achieve the function, as described in detail below. Examples of suitable numbers of followers associated with a particular actuator in a delivery device according to a particular embodiment included, but are not limited to, one, two, more than two, a plurality, three, four, five, six, seven, eight, nine, ten, and more than ten. Furthermore, the same or different numbers of followers can be used with individual actuators in a delivery device according to a particular embodiment. For example, the example delivery device 100 illustrated in Figures 2 and 3A include three followers 510, 530, 550 associated with the first actuator 310, one follower 510' associated with the second actuator 330, and one follower 510" associated with the third actuator 350.

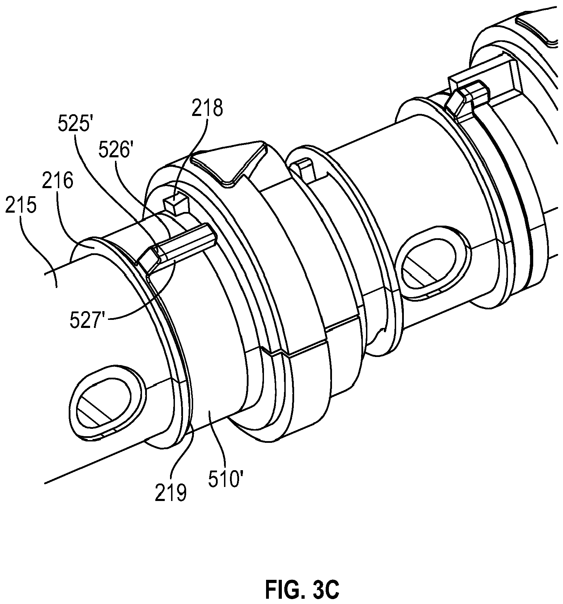

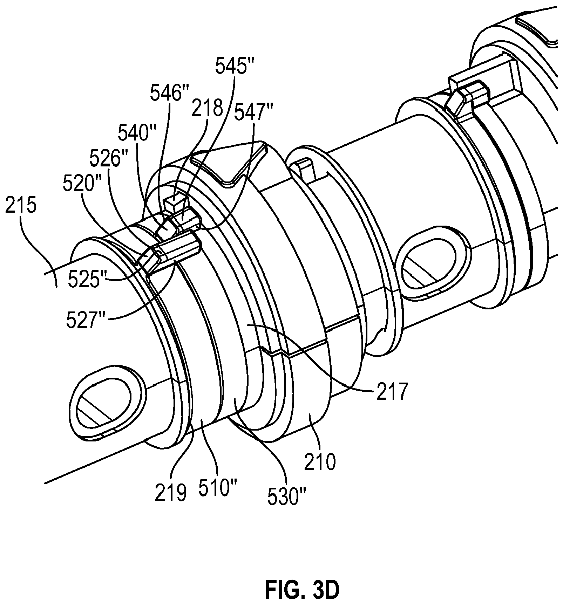

In the example delivery device 100, each of the followers 510, 530, 550 associated with the first actuator 310 has the same axial length. It is noted, though, that followers associated with an individual actuator can have the same or different axial lengths. Indeed, a delivery device according to an embodiment can include multiple channels, each having the same axial length extending between the circumferential walls that define the channel, and sets of followers disposed in each channel as described above. Each set of followers can have any suitable number of followers, as described above, and the followers in each set can have the same or different axial lengths. This structural configuration enables the production of a standard inner handle body with a fixed number of channels, each associated with an actuator of the delivery device and having same axial length extending between its defining circumferential walls. During fabrication of the delivery device, one or more followers of appropriate axial length can be disposed in each individual channel to achieve a desired number of rotations about the inner handle body of the delivery system for the associated actuator to achieve its function. Examples of suitable axial lengths for followers include, but are not limited to, about 1x the channel axial length (one follower disposed in the channel), about 0.5x the channel axial length (two followers disposed in the channel), about 0.33x the channel axial length (three followers disposed in the channel), about 0.25x the channel axial length (four followers disposed in the channel), about 0.2x the channel axial length (five followers disposed in the channel), and about 0.10x the channel axial length (ten followers disposed in the channel). Other axial lengths are contemplated, too, that allow for combination with one or more other followers to fill the axial width of the channel. Examples of additional suitable axial lengths for followers include, but are not limited to, about 0.9x the channel axial length, 0.8x the channel axial length, 0.7x the channel axial length, 0.67x the channel axial length, 0.6x the channel axial length, 0.4x the channel axial length, and about 0.3x the channel axial length. Figure 3C illustrates an example delivery device with a single follower 510' disposed in a channel 219. Follower 510' has an axial length of about 1x the axial length of channel 219. Figure 3D illustrates an example delivery device with followers 510", 530" of different axial lengths disposed in a channel 219. Follower 510" has an axial length of about 0.67x the axial length of channel 219 and follower 530" has an axial length of about 0.33x the axial length of channel 219.

The followers 510, 530, 550 in the illustrated example have the same visual appearance. In some examples, however, followers in a plurality of followers have different visual appearances. For example, each follower in a plurality of followers in a delivery device according to a particular embodiment can have a color that is different from the color of each of the other followers in the plurality of followers. Color-coding of followers in a plurality of followers in this manner can facilitate assembly of a delivery device according to an embodiment, and verification of assembly of a delivery device according to an embodiment. Any suitable color-coding scheme can be used, including a color-coding scheme that only represents different colors for each follower of a plurality of followers and does not signify any particular feature of an individual follower. For example, in a plurality of three followers, one follower could be red, one follower could be blue, and one follower could be green. Each follower could have any suitable physical properties, including axial length and lug arc, as described herein. Alternatively, a color-coding scheme in which individual colors represent one or more physical properties of a follower can be used. For example, in a plurality of two followers having two different axial lengths, one follower having a first axial length could be a first color, such as red, a second follower having a second axial length could be a second color, such as blue. If needed based on the total range of rotation needed and the axial length of the channel within which the plurality of followers is being disposed, a third follower could be added to the plurality. If appropriate based on the total range of rotation needed and that provided by the first and second followers, another of the red or blue followers, with the respective axial length, could be included in the plurality of followers. If an axial length is needed that is different from the axial lengths of the first and second followers, a third follower having a third axial length and a third color, such as green, could be included in the plurality of followers. A similar color-coding scheme can be used for lug arcs. For example, in a plurality of two followers having two different lug arcs, one follower having a first lug arc could be a first color, such as red, a second follower having a second lug arc could be a second color, such as blue. If needed based on the total range of rotation needed, a third follower could be added to the plurality. If appropriate based on the total range of rotation needed, another of the red or blue followers, with the respective lug arc, could be included in the plurality of followers. If a lug arc is needed that is different from the lug arcs of the first and second followers, a third follower having a third lug arc and a third color, such as green, could be included in the plurality of followers.

Inclusion of a plurality of followers having different colors, as described above, can facilitate verification of assembly of a delivery device according to an embodiment. Inclusion of a transparent or semi-transparent inner shell, a transparent or semi-transparent outer shell, or both that is disposed over the plurality of followers during assembly can facilitate inspection of an assembled delivery device having a plurality of followers of different colors, and verification of the inclusion and order of followers in the plurality of followers according to the color-coding scheme, the associated physical properties of the followers, such as axial length, lug arc, or both, and desired properties of the delivery device being assembled.

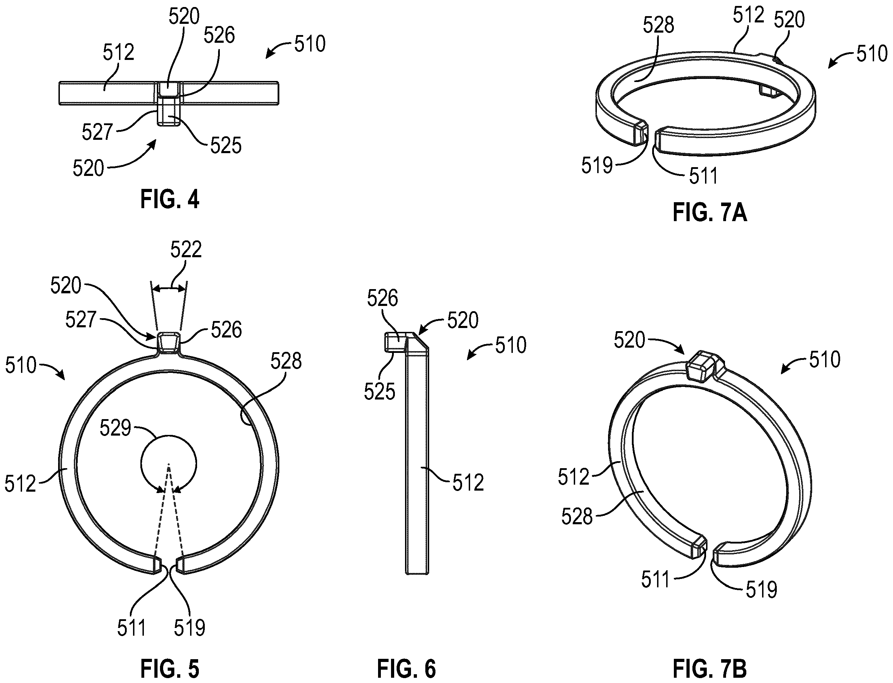

Each of Figures 4, 5, 6, 7A, and 7B illustrates follower 510 isolated from other components of example delivery device 100. All followers in a delivery device according to an embodiment have a similar structure that the structure of illustrated follower 510, but each follower may have a different axial length, as noted above. Follower 510 comprises an arcuate body portion 512 terminating in circumferentially spaced-apart first ends 511 and second ends 519. The first 511 and second 519 ends are resiliently displaceable from each other to allow a snap-fit over an inner handle body of a delivery device according to an embodiment. Follower 510 comprises an arcuate bearing surface 528 that contacts and engages with an inner handle body of a delivery device according to an embodiment for relative rotation about the inner handle body. The bearing surface 528 extends around a bearing arc 529 of at least 190 degrees around the shaft portion as is shown in Figure 5. The length of the arms and the extent to which the bearing surface 528 extends around the shaft portion 215 can be varied to suit the material being used and the particular requirements of the specific application.

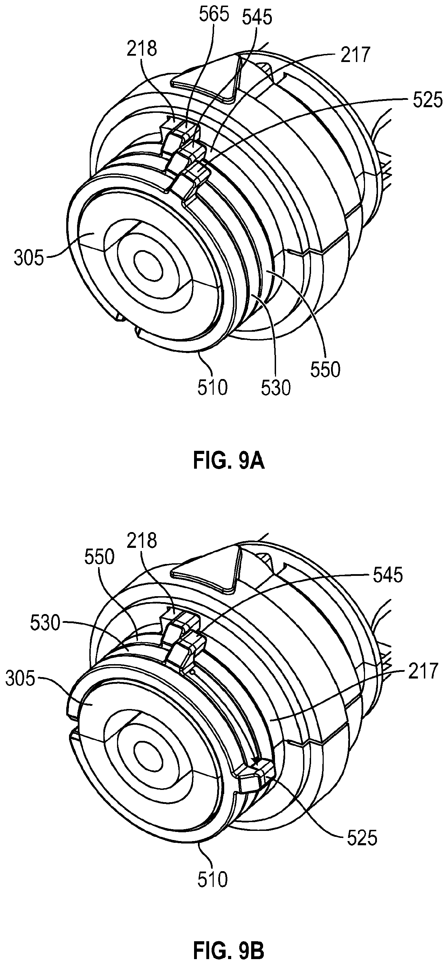

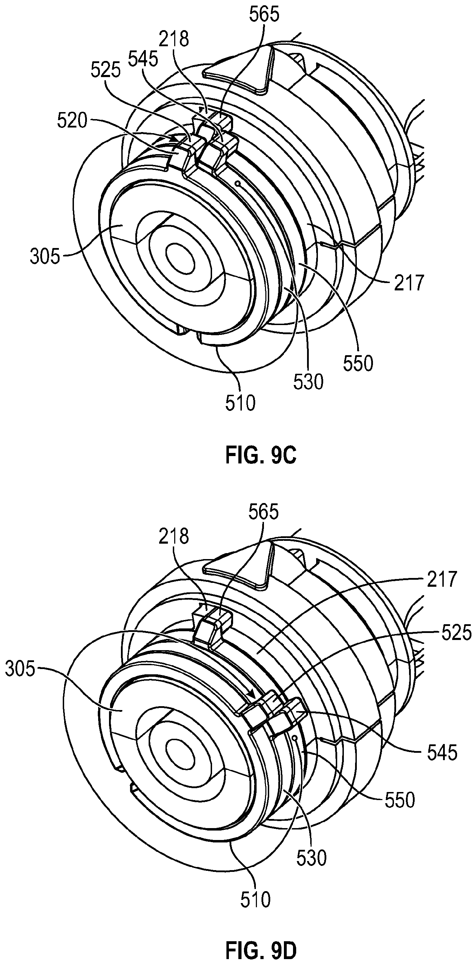

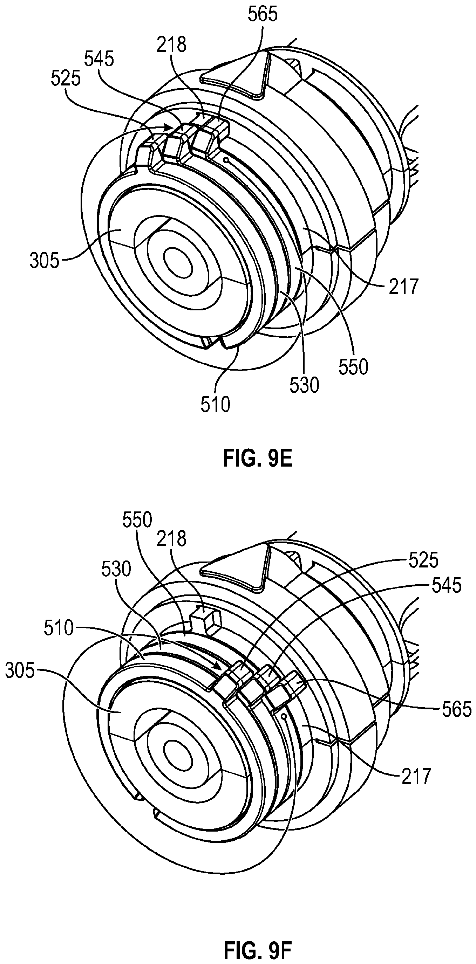

The delivery device 100 further includes a hard stop 218 which is fixed against rotation with respect to the inner handle body 305 as is most clearly shown in Figure 3B. In the illustrated example, hard stop 218 is defined by second circumferential wall 217. The follower-driver portion 322 of the actuator, the plurality of followers 510, 530 and 550, and the hard stop 218 act together to arrest rotation of the actuator 310 with respect to the inner handle body 305 at an end rotation greater than 360 degrees, as is illustrated in the progression of figures 9A to 9G. In the example illustrated, the full rotation of actuator 310 is approximately 1065 degrees.

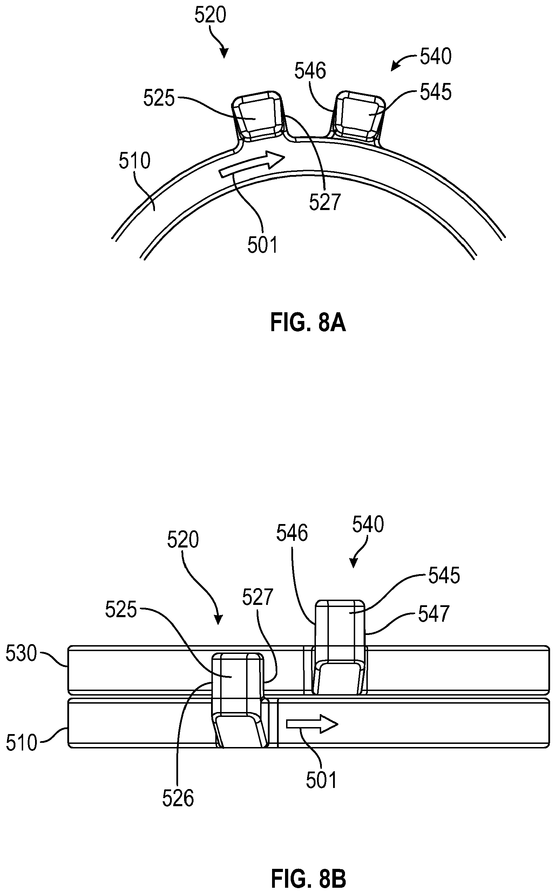

Referring again to Figures 4 to 8B, follower 510 includes a lug portion 520 that extends radially outwards from the body portion 512. As best illustrated in Figure 6, lug portion 520 has an axially extending cantilever portion 525. As best illustrated in Figure 8B with reference to Figure 6, cantilever portion 525 of one follower 510 extends axially over the body portion 512 of an adjacent follower 530, with respect to a longitudinal axis of the followers 510, 530, when followers 510, 530 are situated axially adjacent each other, such as in assembled delivery device 100. Advantageously, the cantilever portion 525 of one follower is nestable over the body portion 512 of an axially adjacent follower, as illustrated in Figures 3B and 8B. Likewise, cantilever portion 545 of lug portion 540 of the follower 530 extends in an axial direction over an adjacent follower 530. This structure allows arresting of the rotation of an actuator with respect to the inner handle body 305 by sandwiching of the lug portions of the followers associated with the actuator, and in particular, the cantilever portions of the followers, between the follower-driver of the actuator and the hard stop on the body of the handle.

Referring to Figures 5, 8A and 8B, each lug portion 520 has a first face 526 and a second face 527. The second face 527 is opposite the first face 526 on the cantilever portion 525 and rotates around the inner handle body 305 as the first face 526 is pushed around the inner handle body 305, by either the follower driver 322 or a second face 527 of an adjacent follower 510 after it has completed its rotation about the inner handle body 305. An arcuate distance between the first face 526 and the second face 527 defines a lug arc 522, as best illustrated in Figure 12. Any lug arc less than 360° can be used for a follower, and a skilled artisan will be able to select an appropriate lug arc for a follower, and for each follower in a plurality of followers, in a delivery device according to a particular embodiment based on various considerations, including a desired total range of rotation of the associated actuator. Examples of suitable lug arcs include, but are not limited to, 12°, 28°, 30°, 45°, 60°, 90°, 180°, and 270°.

Referring to the specific plurality of followers 510, 530, 550 for actuator 310 of the illustrated example delivery device 100, as best illustrated in Figure 3B, first face 526 of first follower 510 is driven by the follower-driver 322 (shown in Figure 3A) on the actuator inner shell 321. First face 546 of second follower 530 is driven by the second face 527 of the first follower 510 once it completes its rotation about the inner handle body 305. First face 566 of third follower 550 is driven by the second face 547 of the second follower 530 once it completes its rotation about the inner handle body 305. Second face 567 of the third follower 530 contacts hard stop 218 once it completes its rotation about the inner handle body 305, preventing further rotation of the actuator 310.

Figures 8A and 8B illustrate two followers 510, 530 in a stage of rotation about the inner handle body (not illustrated in the figures) of the example delivery device. In Figures 8A and 8B, follower 510 is diagrammatically rotating in the direction indicated by arrow 501. The rotation of follower 510 moves its lug portion 520, including cantilever portion 525, into engagement with the lug portion 540 of the adjacent (second) follower 530. The first face 526 of the lug portion 520 on the first follower 510 is driven by engagement with the follower driver 322 of the actuator 310 (as illustrated in Figures 1, 2, and 3A). This causes the lug portion 520 to move in the direction of the arrow 501 until its second face 527 engages with the first face 546 of the lug portion 540 of the second follower 530. After the second face 527 of lug portion 520 of first follower 510 contacts and engages first face 546 of lug portion 540 of second follower 530, continued rotation of actuator 310 causes drives combined rotation of first 510 and second 530 followers in the direction of arrow 501.

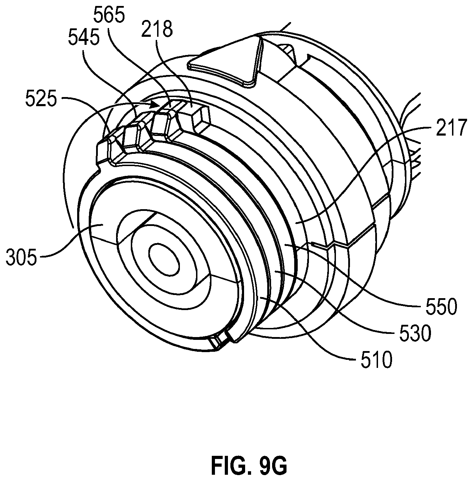

Referring now to Figures 9A and 9C, it can be seen that rotation of the actuator shown in Figures 2 and 3A induces rotation of a first of the plurality of followers, follower 510, though a first follower neutral arc. The first follower neutral arc commences in the position shown in Figure 9A and concludes in the position shown in Figure 9C when the lug portion 520, including cantilever portion 525, of the first follower 510 reaches the lug 540 portion of the second follower 530. What follows is rotation of the first follower 510 through a first follower working arc, during which the second follower 530 of the plurality of followers is rotated by the first follower 510, as is illustrated in the progression from Figure 9C to 9E. At an end of a final working arc, shown in Figure 9G, the second follower 530, or in the illustrated example a subsequent third follower 550, engages the hard stop 218 on the inner handle body 305 thereby stopping any further rotation of the actuator 310.

Returning to Figures 1A, 1B and 2, example delivery device includes three actuators, 310, 330 and 350. The rotational range of actuator 310 is constrained by the arrangement of the three followers 510, 530, 550, as described above. Whereas the rotational range of the actuators 330 and 350 are constrained by the arrangement of single followers 510' and 510" as shown in the magnified view of Figure 3A. In delivery devices according to other embodiments, different numbers and configurations of followers can be used with a follower driver and hard stop to provide a desired total range of rotation for each actuator, as described above.

Some or all of the actuators 310, 330 and 350 may actuate a line puller in delivery device 100. For example, in the illustrated example, actuator 310 actuates a line puller where the line forms diameter-reducing ties within a stent graft 50 as shown in Figure 1B. The arrangement of three followers 510, 530 and 550 described above, allows the line puller to be actuated to retract diameter reducing ties precisely and completely since the total rotation is set to match the required length of retraction.

In the illustrated example delivery device 100, the total range of rotation for actuator 310 is (360° - y°) * z, where y = the lug arc in degrees and z = the number of followers. For the illustrated example, the lug arc 522 is 12° and all followers 510, 530, 550 are identical. Therefore, the total range of rotation for actuator 310 equals (360° - 12°) * 3 = 1044° of total rotation.

The range of rotation for an actuator 510 can be adjusted using different followers, a different number of followers, or a combination of these parameters. For example, if a smaller total range of rotation is desired for a particular actuator, one or more followers with a larger lug arc 522 can be associated with the actuator in a delivery device, such as follower 599 with lug arc 522 of 28° shown in Figure 12. If an actuator includes three identical followers 599, the total range of rotation for the actuator would be (360° - 28°) * 3 = 996°. As another example, if multiple followers with different lug arcs are associated with an actuator, the total range of rotation of the actuator will be the summation of the value of (360° - A°) for all followers associated with the actuator, where A° is the lug arc for a respective follower.

In the illustrated example, the second actuator 330 actuates a line puller where the line is a proximal release wire used to release a proximal end 105 of a stent graft 50. The third actuator 350 actuates a line puller where the line is a distal release wire used to release a distal end of a stent graft 50. The arrangement of followers 510' and 510" shown in Figure 3A, allows the proximal and distal release wires to be precisely and completely controlled since the total range of rotation of each of actuators 330, 350 is set to match the required length of wire retraction for the associated release wire to achieve the desired function.

The hard stop to rotation of an actuator, such as in the examples described and illustrated herein, provides a user of a delivery device according to an embodiment with confidence regarding the status of each actuator in the device.

The structure of the followers 510, 530 and 550 described above facilitates assembly and modification of delivery devices according to embodiments. For instance, in contrast to other delivery device assemblies, it is possible to simply snap on one or more replacement followers having different characteristics, such as the axial length, lug arc, or both. This will be useful in the assembly of delivery devices for custom-made implantable medical devices which may require different actuation lengths for diameter reducing ties, or other structures. Other benefits will also arise from the ease of installation of alternative followers, as described herein.

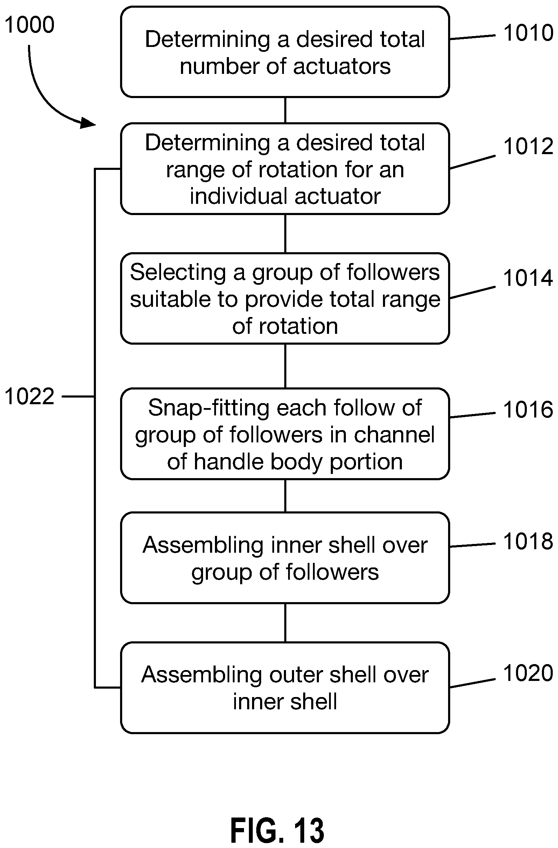

Figure 13 is a flowchart illustration of an example method 1000 of making a delivery device. An initial step 1010 comprises determining a desired total number of actuators. Another step 1012 comprises, for an individual actuator in the desired total number of actuators, determining a desired total range of rotation for the individual actuator. Another step 1014 comprises selecting a group of followers having suitable lug arc and axial length values to provide the desired total range of rotation of the individual actuator. The group of followers can be a single follower or more than one follower, including the example numbers of followers described above. Another step 1016 comprises snap-fitting each follower of the group of followers in a channel of a handle body portion, as described above. Another step 1018 comprises assembling an inner shell over the group of followers such that a follower driver of the inner shell is configured to contact a first face of a first follower of the group of followers. Another step 1020 comprises assembling an outer shell over the inner shell. An optional step 1022 comprises repeating steps 1012, 1014, 1016, 1018, and 1020 for another individual actuator in the desired total number of actuators. Step 1022 can be repeated until each of steps 1012, 1014, 1016, 1018, and 1020 has been performed for all individual actuators in the desired total number of actuators.

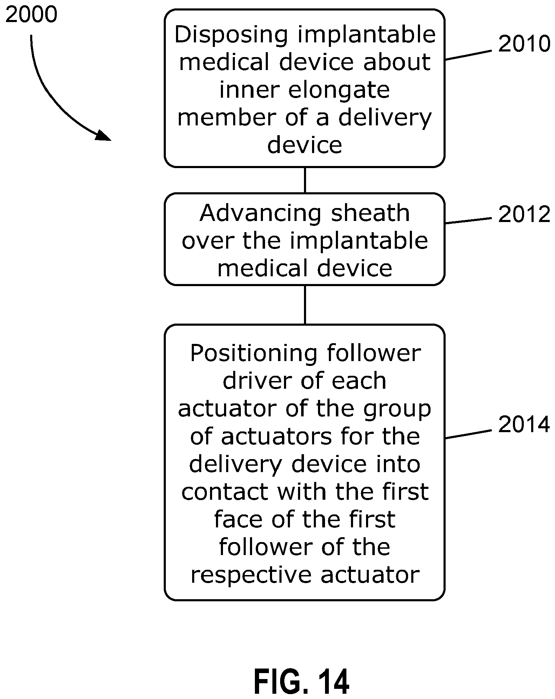

Figure 14 is a flowchart illustration of an example method 2000 of making a delivery system. A step 2010 comprises disposing an implantable medical device about an elongate member of a delivery device according to an embodiment. As an example, the elongate member can be an inner elongate member of a delivery device produced by method 1000 described above and illustrated in Figure 13. An optional step 2012 comprises advancing a sheath of the delivery device over the implantable medical device. Another optional step 2014 comprises positioning the follower driver of each actuator of the group of actuators for the delivery device into contact with the first face of the first follower of the respective actuator.

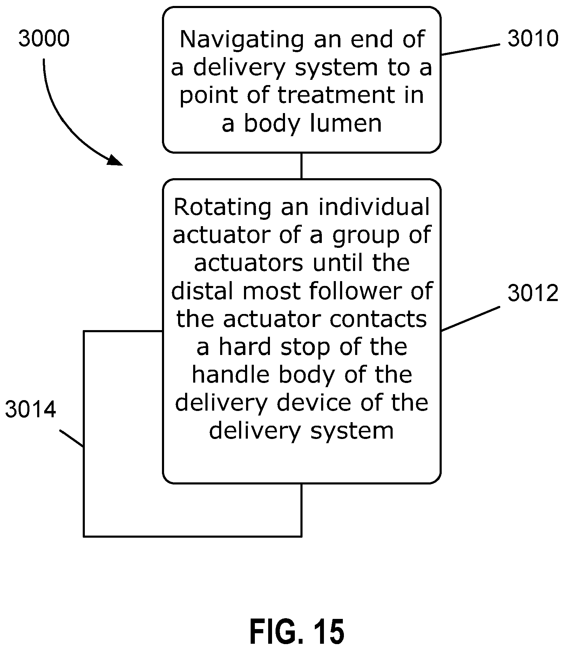

Figure 15 is a flowchart illustration of an example method 3000 of delivering an implantable medical device to a point of treatment in a body lumen. An initial step comprises navigating an end of a delivery system according to an embodiment to a point of treatment in a body lumen. Another step 3012 comprises rotating an individual actuator of a group of actuators in a delivery device of a delivery system according to an embodiment until the second face of the distal most follower of the group of followers for the individual actuator contacts a hard stop of the handle body of the delivery device to arrest rotation of the actuator. An optional additional step 3014 comprises repeating step 3012 for another individual actuator of the group of actuators of the delivery device. Step 3014 is repeated a suitable number of times until the implantable medical device of the delivery system is released from the delivery device of the delivery system at the point of treatment in the body lumen. Step 3014 can be repeated a suitable number of times until step 3012 has been performed for each actuator of the group of actuators.

Throughout this specification and the claims that follow unless the context requires otherwise, the words 'comprise' and 'include' and variations such as 'comprising' and 'including' will be understood to imply the inclusion of a stated integer or group of integers but not the exclusion of any other integer or group of integers.

The reference to any prior art in this specification is not, and should not be taken as, an acknowledgement of any form of suggestion that such prior art forms part of the common general knowledge.

In some cases, a single embodiment may, for succinctness and/or to assist in understanding the scope of the disclosure, combine multiple features. It is to be understood that in such a case, these multiple features may be provided separately (in separate embodiments), or in any other suitable combination. Alternatively, where separate features are described in separate embodiments, these separate features may be combined into a single embodiment unless otherwise stated or implied. This also applies to the claims which can be recombined in any combination. That is a claim may be amended to include a feature defined in any other claim. Further a phrase referring to "at least one of" a list of items refers to any combination of those items, including single members. As an example, "at least one of: a, b, or c" is intended to cover: a, b, c, a-b, a-c, b-c, and a-b-c.

Those with ordinary skill in the art will appreciate that various modifications and alternatives for the described and illustrated examples can be developed based on the overall teachings of the disclosure, and that the various elements and features of one example described and illustrated herein can be combined with various elements and features of another example without departing from the scope of the invention. The particular examples disclosed herein have been selected by the inventors simply to describe and illustrate examples of the invention and are not intended to limit the scope of the invention or its protection, which is to be given the full breadth of the appended claims and any and all equivalents thereof.

The disclosures in Australian patent application number 2022903862, from which this application claims priority, and in the abstract accompanying this application are incorporated herein by reference.

## Claims

1. A delivery device for delivering an implantable medical device into a body lumen of a patient, the delivery device comprising:
a handle assembly having a lengthwise axis and comprising:
a proximal handle portion;
a distal handle portion having a distal handle body defining a circumferential channel and a hard stop adjacent the circumferential channel;
a plurality of followers disposed within the circumferential channel and individually rotatable about the distal handle body, each follower of the plurality of followers having a follower body and a lug having an axially extending cantilever portion with a first face and a second face, the cantilever portion of a first follower of the plurality of followers extending axially over the follower body of an adjacent second follower of the plurality of followers such that a second face of the first follower of the plurality of followers contacts a first face of the second follower of the plurality of followers upon rotation of the first follower about the distal handle body; and
an actuator disposed over the plurality of followers and rotatable about the distal handle body, the actuator defining a follower driver extending radially inward toward the distal handle body and configured to contact the first face of the first follower of the plurality of followers such that rotation of the actuator drives rotation of the first follower of the plurality of followers;
an inner elongate member extending from the handle assembly; and
a sheath disposed over the inner elongate member.

2. A delivery device according to claim 1, wherein each follower of the plurality of followers has an axial length extending along the lengthwise axis of the handle assembly; and
wherein each follower of the plurality of followers has the same axial length; or
wherein each follower of at least two followers of the plurality of followers has an axial length that is different from the axial length of another follower of the plurality of followers; or
wherein each follower of the plurality of followers has an axial length that is different from the axial length of all other followers of the plurality of followers.

3. A delivery device according to claim 1 or 2, wherein each follower of the plurality of followers has a lug arc extending between the first face and the second face of the cantilever portion of the follower; and
wherein each follower of the plurality of followers has the same lug arc; or
wherein each follower of at least two followers of the plurality of followers has a lug arc that is different from the lug arc of another follower of the plurality of followers; or
wherein each follower of the plurality of followers has a lug arc that is different from the lug arc of all other followers of the plurality of followers.

4. A delivery device according to any preceding claim, wherein each follower of the plurality of followers has an axial length extending along the lengthwise axis of the handle assembly;
wherein each follower of the plurality of followers has a lug arc extending between the first face and the second face of the cantilever portion of the follower; and
wherein each follower of the plurality of followers has the same axial length and the same lug arc.

5. A delivery device according to any preceding claim, wherein the follower body of each follower terminates in circumferentially spaced-apart first and second ends.

6. A delivery device according to claim 5, wherein the first and second ends of each follower are resiliently displaceable from each other.

7. A delivery device according to any preceding claim, wherein the plurality of followers comprises two or three followers.

8. A delivery device according to any preceding claim, wherein the distal handle body defines a second circumferential channel and a second hard stop adjacent the second circumferential channel; and
further comprising:
a second plurality of followers disposed within the second circumferential channel and individually rotatable about the distal handle body, each follower of the second plurality of followers having a follower body and a lug having an axially extending cantilever portion with a first face and a second face, the cantilever portion of a first follower of the second plurality of followers nestable over the follower body of an adjacent second follower of the second plurality of followers such that a second face of the first follower of the second plurality of followers contacts a first face of the second follower of the second plurality of followers upon rotation of the first follower about the distal handle body; and
a second actuator disposed over the second plurality of followers and rotatable about the distal handle body, the second actuator defining a second follower driver extending radially inward toward the distal handle body and configured to contact the first face of the first follower of the second plurality of followers such that rotation of the second actuator drives rotation of the first follower of the second plurality of followers.

9. A delivery device according to any preceding claim, wherein the proximal handle portion defines a rounded triangular cross-sectional shape.

10. A delivery device according to any preceding claim, wherein the proximal handle portion comprises a yoke defining a proximal handle portion proximal end and an apron extending to an opening; and
wherein the inner elongate member and sheath extend through the opening.

11. A delivery device according to any preceding claim, wherein the proximal handle portion proximal end lies on a plane disposed at a non-orthogonal angle to the lengthwise axis of the handle assembly.

12. A delivery device according to any preceding claim, wherein the handle assembly comprises a transition portion axially disposed between the proximal handle portion and the distal handle portion.

13. A delivery device according to claim 12, wherein the proximal handle portion has a side length equal to the length of one side of the triangular cross-sectional shape of the proximal handle portion;
wherein the distal handle portion defines a circular cross sectional shape and has a first outer diameter
wherein the transition portion has a second outer diameter that is less than the first outer diameter and less than the side length.

14. A delivery device for delivering an implantable medical device into a body lumen of a patient, the delivery device comprising:
a handle assembly having a lengthwise axis and comprising:
a proximal handle portion defining a rounded triangular cross-sectional shape;
a distal handle portion defining a circular cross-sectional shape, a first circumferential wall defining a hard stop, and a second circumferential wall, a circumferential channel disposed between the first and second circumferential walls;
a first follower disposed in the circumferential channel, the first follower rotatable about the distal handle body and defining a first follower body and a first follower lug having a first follower cantilever portion with a first face and a second face, the first follower cantilever portion extending axially over the first circumferential wall such that the second face of the first follower cantilever portion is configured to contact the hard stop following rotation of the first follower;
a second follower disposed in the circumferential channel, the second follower rotatable about the distal handle body and defining a second follower body and a second follower lug having a second follower cantilever portion with a first face and a second face, the second follower cantilever portion extending axially over the first follower body such that the second face of the second follower cantilever portion is configured to contact the first face of the first follower cantilever portion following rotation of the first follower; and
an actuator disposed over the plurality of followers and rotatable about the distal handle body, the actuator defining a follower driver extending radially inward toward the distal handle body and configured to contact the first face of the first follower of the plurality of followers such that rotation of the actuator drives rotation of the first follower of the plurality of followers;
an inner elongate member extending from the handle assembly; and
a sheath disposed over the inner elongate member.

15. A delivery device for delivering an implantable medical device into a body lumen of a patient, the delivery device comprising:
a handle assembly having a lengthwise axis and comprising:
a proximal handle portion defining a rounded triangular cross-sectional shape;
a distal handle portion defining a circular cross-sectional shape, a circumferential channel, and a hard stop adjacent the circumferential channel;
a follower disposed in the circumferential channel and rotatable about the distal handle body, the follower defining a cantilever portion extending axially over the first circumferential wall configured to contact the hard stop; and
an actuator disposed over the follower and rotatable about the distal handle body, the actuator defining a follower driver extending radially inward toward the distal handle body and configured to contact the follower such that rotation of the actuator drives rotation of the follower; and
an inner elongate member extending from the handle assembly; and
a sheath disposed over the inner elongate member.
